# FASCICULE DE BREVET EUROPEEN

(11) **EP 1 652 570 B1**
(45) Date de publication et mention de la délivrance du brevet: **23.06.2010**
(21) Numéro de dépôt: 05292202.8
(22) Date de dépôt: 19.10.2005
(51) Int. Cl.: B01D 53/04, C07C 7/13, B01D 15/00, B01J 20/18

(54) **Procédé de séparation par adsorption sélective sur un solide contenant une zéolithe de structure cristalline analogue à l'IM-12**
Trennverfahren durch selektive Adsorption mittels eines Festkörpers enthaltend ein Zeolith mit einer IM-12-ähnlichen Kristallstruktur
Separation process by selective adsorption with a solid containing a zeolite having a IM-12-analogous crystalline structure

(30) Priorité: 29.10.2004 FR 0411629
(43) Date de publication de la demande: 03.05.2006
(73) Titulaire: Institut Français du Pétrole, 92852 Rueil Malmaison Cedex (FR)
(72) Inventeur: Leflaive, Philibert, 91440 Bures sur Yvette (FR); Dubreuil, Anne-Claire, 69003 Lyon (FR); Caullet, Philippe, 68440 Illzach (FR); Patarin, Joël, 68720 Flaxlanden (FR); Paillaud, Jean Louis, 68100 Mulhouse (FR)

(56) Documents cités:
- EP-A- 0 820 972
- EP-A- 0 908 229
- EP-A- 1 454 882
- EP-A- 1 518 827
- FR-A- 2 813 310
- US-A- 5 284 992
- US-B1- 6 353 144
- PAILLAUD J.-P. ET AL: 'Extra-Large-Pore Zeolites with Two-Dimensional Channels Formed by 14 and 12 Rings' SCIENCE vol. 304, 14 Mai 2004, pages 990 - 992

## Description

### Domaine de l'invention:

L'invention concerne un procédé de séparation par adsorption utilisant comme masse adsorbante un solide contenant une zéolithe de structure particulière, analogue à celle de l'IM-12.

La séparation par adsorption constitue aujourd'hui une technologie de choix lorsque les technologies basées sur l'équilibre liquide-vapeur, telle que la distillation, ne permettent pas la séparation des différentes espèces d'un mélange.

La séparation par adsorption est largement utilisée pour la séparation et la purification des gaz et des liquides dans des domaines très variés, allant des industries pétrolières, pétrochimiques et chimiques, aux applications environnementales et pharmaceutiques.

Les applications industrielles typiques de la séparation par adsorption sont la production des gaz industriels (oxygène, azote, hydrogène), la séparation des hydrocarbures (paraffines linéaires et branchées, xylènes, par exemple), les traitements de l'air, des eaux et des effluents pour élimination de polluants (composés soufrés, composés organiques volatils...), le séchage, la séparation d'isomères chiraux, etc.

### Examen de l'art antérieur:

Les procédés de séparation par adsorption sont bien connus de l'art antérieur.

Une description sommaire des caractéristiques de ce type de procédés peut être trouvée par exemple dans le volume B3 de l'Encyclopédie Ullmann (Encyclopédie Ullmann) (p9-37 à 9-50) ou dans le volume 4 du « Handbook of porous solids » (dont on peut traduire le titre par « Manuel des solides poreux ») édité chez Wiley and Sons.

Dans l'ensemble des procédés de séparation par adsorption, on peut citer le procédé dit en Contre-Courant Simulé (CCS) décrit par exemple dans les brevets US 2985589 et FR 2681066, le procédé dit « Pressure Swing Adsorption » (PSA ou procédé d'adsorption par variation de pression) décrit par exemple dans les brevets US 6641664, FR 2655980, FR 2837722 ou FR 2774386 et le procédé dit « Thermal Swing Adsorption » (TSA ou procédé d'adsorption par variation de température) décrit par exemple dans les brevets US 6432171 et EP 1226860.

Le principe d'un procédé de séparation par adsorption repose sur l'adsorption sélective d'un ou plusieurs constituants sur un solide microporeux.

Les solides adsorbants peuvent être de plusieurs types, zéolithes ou tamis moléculaires, gels de silice, alumines, charbons actifs.

Tous ces solides sont caractérisés par une grande surface spécifique, de l'ordre de 300 à 1200 m²/g. Les zéolithes se différencient des autres types de solides adsorbants par le fait que ce sont des solides cristallins microporeux et que l'adsorption a lieu à l'intérieur des cristaux. On entend par microporeux une taille de pores inférieure à 20 Å.

Il existe un grand nombre de zéolithes, naturelles ou synthétiques, répertoriées dans « Atlas of Zeolites Structure Types », (« Atlas des structures zéolithiques types ») de Ch. Baerlocher, W.M. Meier et D.H. Olson, 5ième édition revue, 2001, Elsevier, publié par International Zeolite Association (IZA).

Elles se différencient entre elles par leur composition et leur structure cristalline.

La structure cristalline décrit un réseau bidimensionnel ou tridimensionnel de canaux et/ou de pores de taille bien définie qui constitue le volume microporeux.

La taille des ouvertures qui contrôlent l'accès à ces pores est également un paramètre important pour les séparations par adsorption.

Parmi les zéolithes synthétisées depuis une quarantaine d'années, un certain nombre de solides ont permis de réaliser des progrès significatifs dans les domaines de l'adsorption. Parmi celles-ci, on peut citer la zéolithe Y (US 3,130,007) et la zéolithe ZSM-5 (US 3,702,886).

Parmi les zéolithes récemment synthétisées, on peut évoquer l'IM-12 décrite dans la demande de brevet n° EP-A-15 18 827 de la demanderesse et dans J.-L. Paillaud et al., Extra-Large-Pore Zeolites with Two-Dimensional Channels Formed by 14 and 12 Rings, Science, Vol. 304, pages 330-332. Ce solide cristallisé IM-12 présente outre une nouvelle structure cristalline, une composition chimique exprimée sur une base anhydre, en termes de mole d'oxydes, définie par la formule générale suivante : XO₂ : m YO₂ : p Z₂O₃ : q R_{2/n}O dans laquelle R représente un ou plusieurs cation(s) de valence n, X représente un ou plusieurs élément(s) tétravalent(s) différent(s) du germanium, Y représente le germanium, Z représente au moins un élément trivalent.

Les lettres m, p, q, représentent respectivement le nombre de mole de YO₂, Z₂O₃ et

R_{2/n}O, m étant compris entre 0 et 1, p étant compris entre 0 et 0,5, et q étant compris entre 0 et 0,7.

Ce solide cristallisé IM-12 possède en particulier une nouvelle topologie avec un système bidimensionnel de canaux interconnectés, comprenant deux types de canaux droits définis par des ouvertures à 14 et 12 atomes X et/ou Y et/ou Z respectivement, lesdits atomes étant en coordinence 4, c'est-à-dire entourés de quatre atomes d'oxygène.

Le terme « diamètre de pore » est utilisé pour définir de façon fonctionnelle la taille d'un pore en terme de taille de molécule capable d'entrer dans ce pore. Il ne désigne pas la dimension réelle du pore car celle-ci est généralement difficile à déterminer puisque souvent de forme irrégulière (c'est-à-dire le plus souvent non circulaire).

D.W. Breck fournit une discussion sur le diamètre de pore effectif dans son livre intitulé « Zeolite Molecular Sieves » (John Wiley and Sons, New York, 1974) aux pages 633 à 641 dont la traduction française du titre est « Tamis moléculaires zéolithiques ».

Les sections des canaux des zéolithes étant des anneaux d'atomes d'oxygène, on peut également définir la taille des pores des zéolithes par le nombre d'atomes d'oxygène formant la section annulaire des anneaux, désigné par le terme « member rings » ou MR en anglais.

Il est par exemple indiqué dans « Atlas of Zeolites Structure Types », (« Atlas des structures zéolithiques ») de Ch. Baerlocher, W.M. Meier et D.H. Olson, 5ième édition revue, 2001, publié chez Elsevier, que les zéolithes de type structural FAU ont un réseau de canaux cristallins de 12 MR, c'est-à-dire dont la section est constituée de 12 atomes d'oxygène. Le solide cristallisé IM-12 présente quant à lui un réseau bidimensionnel de canaux interconnectés comprenant deux types de canaux droits définis par des ouvertures à 14 et 12 MR. Cette définition est bien connue de l'homme de l'art et sera utilisée dans la suite du texte.

La séparation par adsorption est basée sur une adsorption sélective (soit thermodynamique, soit cinétique) des différents constituants gazeux ou liquides constituant la charge grâce à des interactions spécifiques entre la surface du solide adsorbant et les molécules adsorbées.

Dans la suite du texte, pour simplifier, on parlera d'adsorbant pour désigner le solide adsorbant et de molécule d'adsorbat ou d'adsorbat pour désigner l'espèce adsorbée.

Les séparations par adsorption peuvent être basées sur des effets stériques, cinétiques ou d'équilibre thermodynamique.

Lorsqu'un effet stérique est mis en jeu, seules les molécules dont le diamètre critique est inférieur au diamètre des pores sont adsorbées dans l'adsorbant.

Les différentes espèces contenues dans le mélange sont donc séparées en fonction de la taille moléculaire de ces espèces.

Un exemple typique de ce type de séparation est la séparation des alcanes linéaires et branchés par la zéolithe 5A telle qu'illustrée dans les brevets EP 0820972 et US 6353144 de la demanderesse.

En plus de l'effet stérique, les mélanges d'espèces moléculaires peuvent être séparés par un effet cinétique si l'une des espèces est adsorbée beaucoup plus rapidement ou beaucoup plus lentement que les autres espèces contenues dans le mélange.

La prédominance de l'effet stérique ou de l'effet cinétique dépend de la taille et de la distribution des micropores.

Si le diamètre critique d'une molécule d'adsorbat est de taille comparable à celle des pores de l'adsorbant, un effet stérique et cinétique peut se produire car les molécules d'adsorbat plus petites peuvent s'adsorber plus rapidement. Un tel effet se produit par exemple dans la séparation des paraffines multibranchées sur un adsorbant zéolithique de structure mixte avec des canaux principaux ayant une ouverture à 10 MR et des canaux secondaires ayant une ouverture à au moins 12 MR tel qu'illustrée dans le brevet FR 2813310 de la demanderesse.

Ce brevet décrit un procédé de séparation des paraffines multibranchées d'une charge hydrocarbonée contenant des hydrocarbures ayant de 5 à 8 atomes de carbone par molécule, notamment des paraffines linéaires, monobranchées et multibranchées en utilisant une zéolithe de type structural NES (zéolithe NU-85 ou NU-86 par exemple).

Les séparations par adsorption basées sur des effets d'équilibre thermodynamique reposent sur l'adsorption préférentielle d'un des composés par rapport aux autres composés contenus dans le mélange à séparer. Dans le cas de ces séparations dites « thermodynamiques », l'adsorbant a un diamètre de pores supérieur au diamètre critique des molécules à séparer, en fait le plus large possible, de manière à faciliter la diffusion macroporeuse des molécules. Un exemple de ce type de séparation est la séparation du paraxylène à partir d'une charge contenant des xylènes et de l'éthylbenzène sur des zéolithes de type Faujasite dont l'arrière-plan technologique est illustré dans le brevet EP0531191 de la demanderesse.

Une des caractéristiques essentielles de la technologie d'adsorption réside dans son fonctionnement transitoire et généralement cyclique puisque, après une phase d'adsorption, le solide adsorbant doit être régénéré partiellement ou complètement pour une prochaine utilisation, c'est-à-dire qu'il doit être débarrassé des espèces adsorbées généralement au moyen d'un solvant de désorption ou par un abaissement de pression (cas des procédés PSA) ou par un effet de température (cas des procédés TSA).

Ce fonctionnement dynamique conduit à une certaine complexité des procédés d'adsorption en terme d'équipements, de contrôle des procédés, de dimensionnement et d'optimisation des durées de cycle d'adsorption et de désorption.

Les performances de séparation dépendent en effet non seulement des propriétés thermodynamiques, mais également des propriétés cinétiques et hydrodynamiques.

Il convient en particulier pour l'adsorbant de posséder un volume poreux le plus important possible et une taille de pore adaptée en fonction du type de séparation désiré.

### Description sommaire des figures:

La figure 1 représente l'isotherme d'adsorption d'azote à 77K du silicogermanate IM-12 synthétisé selon la méthode présentée dans l'exemple 1.

La figure 2 donne une représentation chromatographique de la séparation du paraxylène à partir d'un mélange paraxylène/orthoxylène à 150°C, avec le même silicogermanate IM-12 et un désorbant constitué de toluène pur.

### Description sommaire de l'invention:

L'objet de l'invention concerne un ensemble de procédés de séparation par adsorption en lit mobile simulé d'une espèce moléculaire à partir d'un mélange d'hydrocarbures contenant ladite espèce et d'autres espèces moléculaires utilisant un adsorbant caractérisé en ce qu'il contient un solide de structure cristalline analogue à celle du solide IM-12 et présentant une composition chimique exprimée sur une base anhydre, en termes de mole d'oxydes, par la formule XO₂ : m YO₂ : p Z₂O₃ : q R_{2/n}O, où R représente un ou plusieurs cation(s) de valence n, X représente un ou plusieurs élément(s) tétravalent(s) différent(s) du germanium, Y représente le germanium et Z représente au moins un élément trivalent.

Le mélange contenant l'espèce moléculaire à séparer peut être un mélange d'hydrocarbures quelconque, au sens où les nombres d'atomes de carbone de chaque espèce formant le mélange peuvent être quelconque.

L'invention peut s'appliquer dans des domaines très variés, allant des industries pétrolières, pétrochimiques et chimiques, aux applications environnementales et pharmaceutiques.

Un procédé de type contre-courant simulé fonctionne à une température souvent fixée entre 20°C et 250°C environ, et de préférence entre 60°C et 210°C. La pression est supérieure à la pression de bulle des espèces à séparer afin de maintenir la phase liquide dans l'ensemble du système. Le rapport volumique du désorbant sur la charge est généralement compris entre 0,5 et 30.

Si l'une des espèces moléculaires est une impureté, c'est-à-dire typiquement à une concentration inférieure à 1% poids, et plus particulièrement compris entre 0,1% poids et quelques ppm poids, le procédé selon l'invention peut se réduire au passage du flux à traiter à travers un ou plusieurs lits d'adsorbants dans une gamme de température comprise entre -50°C et 300°C, de préférence entre -50°C et 100°C. La régénération du ou des lits peut s'effectuer en utilisant un gaz de purge qui traverse le ou les lits dans une gamme de température comprise entre -50°C et 300°C, de préférence entre -50°C et 150°C de manière à désorber l'impureté de l'adsorbant.

L'adsorbant sera adapté en fonction de l'application visée. Ainsi, plusieurs paramètres tels que le rapport X/Ge, le rapport (X+Ge)/Z, la nature du ou des cation(s) R seront ajustés afin d'assurer une performance optimale du procédé. De la même manière, la forme sous laquelle sera utilisée l'adsorbant (extrudés, poudre, billes) dépendra du type de procédé utilisé.

### Exposé détaillé de l'invention:

L'objet de l'invention est un ensemble de procédés de séparation par adsorption, qu'on appellera de manière générique procédé de séparation, utilisant un adsorbant caractérisé en ce qu'il contient un solide de structure cristalline analogue à celle du solide IM-12 et présentant une composition chimique exprimée sur une base anhydre, en termes de mole d'oxydes, par la formule XO₂ : m YO₂ : p Z₂O₃ : q R_{2/n}O, où R représente un ou plusieurs cation(s) de valence n, X représente un ou plusieurs élément(s) tétravalent(s) différent(s) du germanium, Y représente le germanium et Z représente au moins un élément trivalent. La demande de brevet EP-A-15 18 827 décrit la zéolithe IM-12 et son procédé de séparation.

Par rapport à l'art antérieur, le procédé selon l'invention présente plusieurs avantages:
- Dans le cas où l'effet recherché est un effet stérique et/ou cinétique, l'utilisation d'une zéolithe à larges pores permet de séparer des molécules de taille importante. Cela était déjà envisageable avec d'autres zéolithes telle que la CIT-5 (US-6,043,179), la SSZ-53 ou la SSZ-59 (Burton A. et al, Chemistry: a Eur. Journal, submitted), la OSB-1, la UTD-1F (Wessels T., Baerlocher C., McCusker L.B., Creyghton E.J., J. Am. Chem. Soc. 121, 6242-6247 (1999)), ou l'AIPO-8 (Dessau R.M., Schlenker J.L., Higgins J.B., Zeolites, 10, 522-524 (1990)), mais ces dernières présentent un volume poreux nettement inférieur à celui de l'IM-12. De plus, l'IM-12 est la seule de ces zéolithes à présenter un système bidimensionnel de canaux dont le canal de plus petit diamètre est supérieur à 8 MR.
- Dans le cas où la séparation est basée sur l'équilibre thermodynamique, le procédé selon l'invention présente l'avantage d'offrir une grande qualité de séparation, le solide IM-12 ayant à la fois une grande capacité et de larges canaux droits définis par des ouvertures à 14 et 12 MR formant un système bidimensionnel de canaux interconnectés. Ce système bidimensionnel de canaux de grande taille interconnectés permet en effet une bonne diffusion des espèces moléculaires à l'intérieur de la porosité, réduisant ainsi les résistances diffusionnelles des espèces moléculaires adsorbées.
- Enfin, dans tous les cas, le solide IM-12 présente une haute stabilité thermique, ce qui est primordial afin d'éviter la dégradation du solide utilisé, particulièrement dans les procédés de type TSA. En effet, le solide IM-12 peut résister à plusieurs cycles de calcination à 600°C, température nettement supérieure à celles généralement rencontrées dans les procédés de séparation par adsorption (typiquement 400°C max.)

La structure cristalline du solide cristallisé IM-12 est une structure tridimensionnelle formée de tétraèdres. Elle comprend notamment des unités de type double cycles à quatre tétraèdres. Le sommet de chaque tétraèdre est occupé par un atome d'oxygène. Le solide cristallisé IM-12 possède une nouvelle topologie avec un système bidimensionnel de canaux interconnectés comprenant deux types de canaux droits définis par des ouvertures à 14 et 12 atomes X et/ou Y et/ou Z respectivement, lesdits atomes étant en coordinence 4, c'est-à-dire entourés de quatre atomes d'oxygène.

Les dimensions de ces canaux sont respectivement 9,5 x 7,1 Å pour les canaux à 14 MR et 8,5 x 5,1 Å pour les canaux à 12 MR.

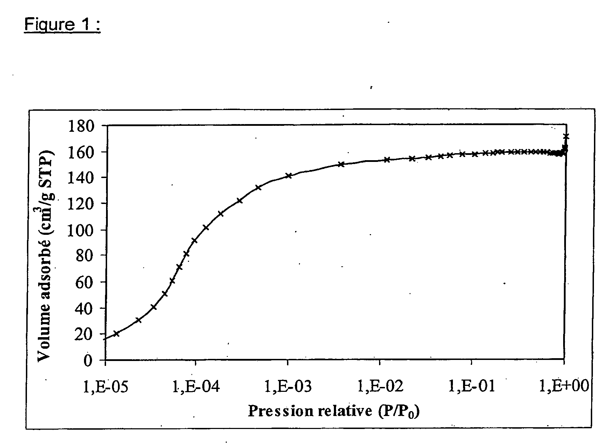

L'isotherme d'adsorption d'azote à 77K du silicogermanate IM-12 illustrée figure 1 est caractéristique d'un matériau purement microporeux de type « la » suivant la nomenclature IUPAC indiquant l'absence de micropores secondaires et de mésoporosité. Le volume microporeux est de 0,26 cm³/g et la surface BET est de 670 m²/g.

A titre de comparaison, les zéolithes de type Faujasite qui sont parmi les zéolithes ayant les plus grands volumes microporeux et les plus grandes ouvertures de pores, ont un volume microporeux de 0,35 cm³/g environ mesuré par adsorption d'azote à 77K, et des diamètres de fenêtres de 7,4 x 7,4 Å (« Atlas of Zeolites Structure Types », Ch. Baerlocher, W.M. Meier et D.H. Olson, 5ième édition revue, 2001, Elsevier, ouvrage déjà cité). Il est à noter que dans le cas des Faujasites, une partie du volume poreux (les cages sodalites) n'est pas accessible aux molécules autres que l'eau et l'azote. Ainsi, par exemple, le volume poreux accessible à un alcane multibranché tel que le 2,2,4-triméthylpentane est de 0,27 cm³/g.

L'ensemble des procédés de séparation par adsorption visant à séparer un produit ou un ensemble de produits à partir d'une charge les contenant sont concernés par la présente invention.

L'invention peut donc s'appliquer dans des domaines très variés, allant des industries pétrolières, pétrochimiques et chimiques, aux applications environnementales et pharmaceutiques.

Plus particulièrement, les applications visées sont la production des gaz industriels, la séparation des hydrocarbures et l'élimination de polluants (composés soufrés, composés organiques volatils...).

De manière préférée, les séparations concernées par la présente invention sont :
- La séparation d'un des isomères du xylène (ortho-, méta- ou para-xylène) ou de l'éthylbenzène à partir d'une charge d'hydrocarbures comprenant essentiellement des hydrocarbures aromatiques en C8. Le désorbant préféré est en général le toluène, cependant d'autres désorbants tels que le paradiéthylbenzène, le paradifluorobenzène ou des diéthylbenzènes en mélange peuvent également convenir.
   Préférentiellement, le rapport volumique du désorbant sur la charge sera compris entre 0,5 et 2,5 et de préférence entre 1 et 2.
   La température sera généralement comprise entre 20°C et 250°C, de préférence entre 90°C et 210°C et plus particulièrement entre 160°C et 200°C et sous une pression comprise entre la pression atmosphérique et 20 bars (1 bar = 0,1 MPa).
- La séparation des paraffines linéaires à partir d'un mélange d'hydrocarbures quelconque les contenant. La température de fonctionnement de l'unité sera préférentiellement comprise entre 100°C et 250°C. La pression dans l'unité sera préférentiellement comprise entre 0,2 et 2 MPa. Le désorbant utilisé sera préférentiellement un hydrocarbure, et en particulier une paraffine en C3-C6 ou un mélange de paraffines en C3-C6.
- La séparation des paraffines linéaires et monobranchées d'une part, et des paraffines multibranchées d'autre part à partir d'un mélange les contenant. La température de fonctionnement de l'unité sera préférentiellement comprise entre 100°C et 250°C. La pression dans l'unité sera préférentiellement comprise entre 0,2 et 2 MPa. Le désorbant utilisé sera préférentiellement un hydrocarbure, et en particulier une paraffine en C3-C6 ou un mélange de paraffines en C3-C6.
- La séparation d'un ou de plusieurs des isomères du diméthylnaphtalène (par exemple le 2,6-diméthylnaphtalène) à partir d'une charge d'hydrocarbures comprenant essentiellement des hydrocarbures aromatiques en C12.
   Le désorbant préféré est généralement le toluène, cependant d'autres désorbants tels que le paradiéthylbenzène, le paradifluorobenzène ou des diéthylbenzènes en mélange peuvent également convenir. Préférentiellement, le rapport volumique de désorbant sur charge sera compris entre 0,5 et 2,5, et de préférence entre 1 et 2. La température sera généralement comprise entre 20°C et 300°C, de préférence entre 90°C et 260°C, et plus particulièrement entre 160°C et 250°C et sous une pression comprise entre la pression atmosphérique et 2 MPa, de préférence 0,2 à 2 MPa.
- La séparation d'une ou de plusieurs oléfines à partir d'une charge d'hydrocarbures comprenant essentiellement des oléfines ou essentiellement des paraffines et des oléfines (par exemple, la séparation du 1,3-butadiène d'un mélange 1,3-butadiène, isobutène, n-butane, isobutane, cis- et trans-2-butènes, la séparation éthane/éthylène, la séparation propane/propylène ou encore la séparation de l'isoprène d'un mélange d'oléfines en C5).
- La séparation d'un des isomères du dichlorobenzène (ortho-, méta- ou paradichlorobenzène) à partir d'une charge comprenant essentiellement des dichlorobenzènes. Le désorbant préféré est généralement le toluène, cependant d'autres désorbants tels le paraxylène, le métaxylène ou des xylènes en mélange peuvent également convenir. La température sera généralement comprise entre 20°C et 250°C, de préférence entre 90°C et 210°C, et plus particulièrement entre 120°C et 200°C et sous une pression comprise entre la pression atmosphérique et 2 MPa, de préférence 0,2 à 2 MPa.
- La séparation des composés aromatiques lourds (« polynuclear aromatics » ou PNA en anglais) présents dans les résidus d'hydrocraquage. On utilisera de préférence plusieurs lits placés en parallèle ou en série. La température et la pression pendant la phase d'adsorption seront préférentiellement choisies afin de maintenir les hydrocarbures en phase liquide. La température sera généralement comprise entre 20°C et 350°C, et plus particulièrement entre 50°C et 250°C et sous une pression comprise entre la pression atmosphérique et 4 MPa, de préférence 0,2 à 4 MPa.
- La purification d'un flux d'hydrocarbures contenant des impuretés soufrées et/ou azotées (par exemple, la désulfuration d'un gazole ou d'une essence). De manière préférée, le flux sera préalablement hydrotraité afin d'abaisser la teneur en composés soufrés et/ou azotés à moins de 500 ppm, et idéalement à moins de 50 ppm. Lors de la phase d'adsorption, la température sera généralement comprise entre 20°C et 400°C, de préférence entre 100°C et 280°C, et plus particulièrement entre 150°C et 250°C et sous une pression comprise entre 0,3 et 3 MPa.

L'adsorbant sera adapté en fonction de l'application visée. Ainsi, plusieurs paramètres tels que le rapport X/Ge, le rapport (X+Ge)/Z, la nature du ou des cation(s) R seront ajustés afin d'assurer une performance optimale du procédé. De la même manière, la forme sous laquelle sera utilisée l'adsorbant (extrudés, poudre, billes) dépendra du type de procédé utilisé.

### Exemples:

L'invention sera mieux comprise à la lecture des exemples suivants qui illustrent l'invention sans toutefois en limiter la portée.

L'exemple 1 présente une séparation par adsorption basée sur un effet stérique et cinétique.

L'exemple 2 concerne la séparation des xylènes.

L'exemple 3 présente la mise en oeuvre du procédé de séparation de l'orthoxylène à partir d'un mélange de xylènes et d'éthylbenzène.

### Exemple 1:

La réaction d'hydrocraquage crée des composés aromatiques lourds indésirables (HPNA, acronyme de Heavy PolyNuclear Aromatics) qui encrassent les équipements et réduisent la durée de vie du catalyseur. Leur taux de formation augmente avec la conversion et le poids moléculaire moyen de la charge.

De manière générale, un recyclage de la fraction non-convertie est nécessaire en sortie du réacteur. Au cours de l'opération, les composés aromatiques lourds s'accumulent dans ce recyclage. Cette accumulation conduit à un encrassement d'autant plus fort du réacteur. Or, seuls les composés les plus lourds génèrent ces problèmes. On a donc intérêt à les retirer du flux de recyclage à l'aide d'un procédé de séparation. L'IM-12 avec ses pores très larges est un adsorbant de choix pour cette séparation.

La synthèse d'un silicogermanate IM-12 a été réalisée selon l'exemple 1 de la demande de brevet EP-A-15 18 827 de la demanderesse. Elle consiste à mélanger dans un bécher :
- 5,78g de solution aqueuse à 20% d'hydroxyde de (6R,10S)-6,10-dimethyl-5-azoniaspiro[4.5]décane (ROH),
- et 0,872g d'oxyde de germanium amorphe (Aldrich),
- puis après dissolution sous agitation de l'oxyde, à ajouter 2,5g de silice colloïdale (Ludox HS-40 (Aldrich)) et 6,626g d'eau.
Après homogénéisation, le gel ainsi obtenu est placé dans un autoclave et chauffé pendant 6 jours à 170°C, sous agitation. Après filtration, le produit est lavé à l'eau distillée et séché à 70°C. L'échantillon est ensuite calciné dans un four à moufles, sous flux constant d'air et jusqu'à une température maximale de 550°C.

On obtient alors le silicogermanate IM-12 sous sa forme calcinée, de formule SiO₂ : 0,23 GeO₂.

Le tableau 1 ci-dessous présente les diamètres cinétiques de différentes molécules contenant un ou plusieurs noyaux aromatiques calculés par Henry W. Haynes Jr., Jon F. Parcher et Norman E. Heimer (Ind. Eng. Chem. Process Des. Dev., 22, 401-409 (1983)).

**Tableau 1**

| **Molécules** | **Nombre de noyaux aromatiques** | **Diamètres critiques** **(Å)** |
|---|---|---|
| Benzène | 1 | 6,7 |
| Naphtalène | 2 | 7,3 |
| Anthracène | 3 | 7,3 |
| Phénanthrène | 3 | 7,8 |
| Pyrène | 4 | 9,0 |
| Coronène | 6 | 11,4 |

Or les dimensions des canaux de l'IM-12 sont respectivement 9,5 x 7,1 Å pour les canaux à 14 MR et 8,5 x 5,1 Å pour les canaux à 12 MR. Une séparation par adsorption basée sur un effet stérique et cinétique permet donc d'isoler les produits de poids moléculaire supérieur ou égal à celui du coronène, tels que l'ovalène (8 noyaux aromatiques), leurs dérivés alkylés, les dimères du coronène, et plus généralement toute molécule de diamètre moléculaire supérieur à celui du coronène.

Pour cette séparation, l'adsorbant est placé dans plusieurs lits fixes disposés en parallèle. La température pendant la phase d'adsorption est comprise entre 50°C et 250°C et la pression est comprise entre la pression atmosphérique et 4 MPa.

### Exemple 2:

Pour cet exemple, nous avons réalisé un test de perçage (test 1) (chromatographie frontale) pour évaluer la capacité de l'IM-12 à séparer l'orthoxylène des autres xylènes.

La synthèse de l'IM-12 a été réalisée selon la méthode décrite dans l'exemple 1.

L'adsorbant est ensuite placé dans une colonne. La quantité utilisée pour chaque test est de 2,63g. Pour chaque test, la température de la colonne est maintenue à 150°C et la pression est suffisante afin de s'assurer que l'on reste en phase liquide, soit environ 1 MPa. Le désorbant utilisé est le toluène.

L'effluent de la colonne est échantillonné (30 échantillons) puis analysé par chromatographie en phase gazeuse afin de déterminer la composition de l'effluent à différents intervalles de temps.

Dans un premier test, la composition de la charge est :
Paraxylène : 45% poids
Métaxylène : 45% poids
Iso-octane : 10% poids (celui-ci est utilisé comme traceur pour l'estimation des volumes non-sélectifs et n'intervient pas dans la séparation)

Dans un second test (test 2), la composition de la charge est :
Paraxylène : 50% poids
Orthoxylène : 50% poids

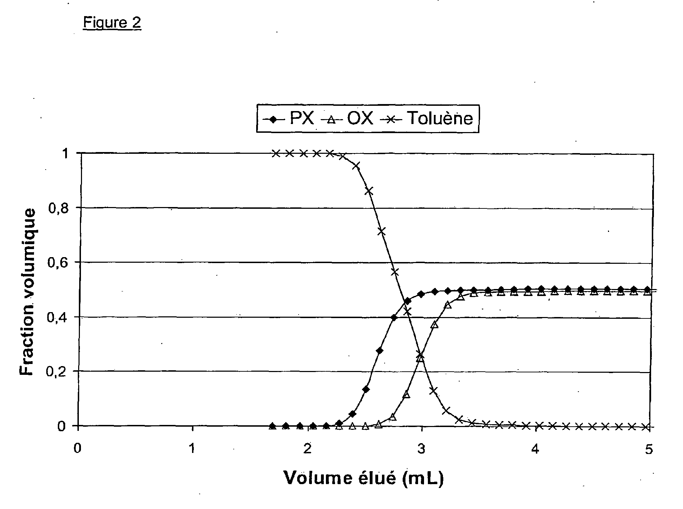

La courbe de perçage obtenue correspondant à cette charge est donnée à la figure 2.

Le mode opératoire utilisé est le suivant :
- Remplissage de la colonne par le tamis et mise en place dans le banc de test.
- Remplissage par le solvant à température ambiante.
- Montée progressive à 150 °C sous flux de toluène (0,2 cm³/min).
- Permutation solvant/charge pour injecter la charge (0,2 cm³/min).
- L'injection de la charge est ensuite maintenue un temps suffisant pour atteindre l'équilibre thermodynamique.
- Collecte et analyse de l'effluent.

La capacité du tamis et sa sélectivité sont ensuite calculées et données dans le tableau suivant. La sélectivité α_{OX/PX} est calculée à partir du test 2. Le test 1 permet de calculer la sélectivité α_{PX/MX.} La sélectivité α_{OX/MX} est calculée comme le produit des deux précédentes.

| Nature du solide | Capacité (g de C8 ads. / g de tamis) | Sélectivité | | Référence |
|---|---|---|---|---|
| | | α_{OX/PX} | α_{OX/MX} | |
| IM-12 | 0,136 | 2,05 | 2,11 | Selon l'invention |
| CSZ-1 (forme K) | 0,12 | 1,4 | 2,4 | US 4,376,226 |
| CSZ-1 (forme Pb²⁺) | 0,08 | 2,1 | 2,8 | US 4,376,226 |
| AlPO₄-5 | 0,057 | 2,6 | 2,7 | US 4,482,776 |
| NaX | * | 1,8 | 1,4 | US 4,482,777 |
| CaY | * | 2,4 | 1,8 | US 4,482,777 |
| AgX | * | 1,81 | 1,64 | US 4,529,828 |

| | | | | |
|---|---|---|---|---|
| * : Contrairement à la chromatographie frontale (courbes de perçage), les expériences en pulses réalisées dans ces références ne permettent pas de calculer la capacité des tamis. | | | | |

Par rapport aux autres adsorbants, on observe que l'IM-12 permet d'obtenir des résultats satisfaisants pour la séparation de l'orthoxylène.

La zéolithe présentant les performances les plus proches est la zéolithe CSZ-1 échangée au plomb. Il est clair que la présence de métaux lourds tels que le plomb est à éviter pour des questions environnementales. Par ailleurs, dans tous les cas, l'IM-12 présente une porosité plus large que les autres adsorbants, ce qui permet une meilleure diffusion des molécules à l'intérieur de la porosité et donc une résistance au transfert de matière réduite.

### Exemple 3:

Une production d'orthoxylène à partir d'une charge comportant un mélange de xylènes et d'éthylbenzène de composition pondérale suivante :
Paraxylène : 1,0% poids
Métaxylène : 63,8% poids
Orthoxylène : 28,0% poids
Ethylbenzène : 7,2% poids
est réalisée en lit mobile simulé, à contre-courant, l'unité étant composée de 24 lits équivalents, chaque lit ayant un volume de 381cm³ et contenant de l'IM-12 réalisée selon la méthode présentée dans l'exemple 1 et mis en forme sous forme de billes. Le solvant utilisé est le toluène.

La température opératoire est 150°C, la pression à l'aspiration de la pompe de recyclage est maintenue à 1 MPa. Tous les flux injectés ou soutirés sont sous contrôle de débit, à l'exception du raffinat sous contrôle de pression.

On compte 5 lits entre l'injection de désorbant et le soutirage d'extrait, 9 lits entre le soutirage d'extrait et l'injection de charge, 7 lits entre l'injection de charge et le soutirage de raffinat, et 3 lits entre le soutirage de raffinat et l'injection de désorbant. Les débits d'injection et de soutirage sont les suivants :
Charge : 25,2 cm³.min⁻¹
Solvant : 37,8 cm³.min⁻¹ de toluène
Extrait : 12,0 cm³.min⁻¹
Raffinat : 51,0 cm³.min⁻¹
Débit de recyclage (en zone 1) : 134 cm³.min⁻¹

Le temps de permutation des vannes (ou période) est de 140 secondes.

La composition de l'extrait est la suivante :
Paraxylène : 0,01% poids
Métaxylène : 0,24% poids
Orthoxylène : 55,76% poids
Ethylbenzène : 0,03% poids
Toluène : 43,96% poids

La composition du raffinat est la suivante :
Paraxylène : 0,49% poids
Métaxylène : 31,47% poids
Orthoxylène : 0,72% poids
Ethylbenzène : 3,55% poids
Toluène : 63,77% poids

Après distillation du toluène, l'extrait obtenu délivre de l'orthoxylène à 99,5% de pureté et un rendement de 94,8%.

## Revendications

1. Procédé de séparation par adsorption en lit mobile simulé d'une espèce moléculaire à partir d'un mélange d'hydrocarbures contenant la dite espèce et d'autres espèces moléculaires en proportion quelconque, comprenant une mise en contact du mélange avec un solide adsorbant, l'adsorbant étant **caractérisé en ce qu'**il contient un solide de structure cristalline analogue à celle de l'IM-12 et présentant une composition chimique exprimée sur une base anhydre et en termes de mole d'oxydes, par la formule XO₂ : m YO₂ : p Z₂O₃ : q R_{2/n}O, où R représente un ou plusieurs cation(s) de valence n, X représente un ou plusieurs élément(s) tétravalent(s) différent(s) du germanium, Y représente le germanium et Z représente au moins un élément trivalent.

2. Application du procédé de séparation par adsorption selon la revendication 1 à la séparation d'un des isomères du xylène (ortho-, méta- ou para-xylène) ou de l'éthylbenzène à partir d'une charge d'hydrocarbures comprenant essentiellement des hydrocarbures aromatiques en C8, le désorbant utilisé étant le toluène, et le rapport volumique du désorbant sur la charge étant compris entre 0,5 et 2,5 et de préférence entre 1 et 2, la température étant comprise entre 20°C et 250°C, de préférence entre 90°C et 210°C, et plus particulièrement entre 160°C et 200°C, et la pression étant comprise entre la pression atmosphérique et 2 MPa.

3. Application du procédé de séparation par adsorption selon la revendication 1 à la séparation des paraffines linéaires à partir d'un mélange d'hydrocarbures quelconque les contenant, la dite séparation étant réalisée en phase liquide, la température étant comprise entre 100°C et 250°C, et la pression étant comprise entre 0,2 et 2 MPa, le désorbant utilisé étant préférentiellement un hydrocarbure, et en particulier une paraffine en C3-C6 ou un mélange de paraffines en C3-C6.

4. Application du procédé de séparation par adsorption selon la revendication 1 à la séparation d'un ou de plusieurs des isomères du diméthylnaphtalène, à partir d'une charge d'hydrocarbures comprenant essentiellement des hydrocarbures aromatiques en C12, le désorbant préféré étant le toluène, le rapport volumique du désorbant sur la charge étant compris entre 0,5 et 2,5, et de préférence entre 1 et 2, la température étant généralement comprise entre 20°C et 300°C, de préférence entre 90°C et 260°C, et plus particulièrement entre 160°C et 250°C, et la pression étant comprise entre la pression atmosphérique et 2 MPa.

5. Application du procédé de séparation par adsorption selon la revendication 1 à la séparation d'un des isomères du dichlorobenzène (ortho-, méta- ou para-dichlorobenzène) à partir d'une charge comprenant essentiellement des dichlorobenzènes, le désorbant préféré étant le toluène, le paraxylène, le métaxylène ou des xylènes en mélange, la température étant comprise entre 20°C et 250°C, de préférence entre 90°C et 210°C, et plus particulièrement entre 120°C et 200°C, et la pression étant comprise entre la pression atmosphérique et 2 MPa.

6. Application du procédé de séparation par adsorption selon la revendication 1 à la séparation des composés aromatiques lourds (« polynuclear aromatics » ou PNA) présents dans les résidus d'hydrocraquage, la température étant généralement comprise entre 20°C et 350°C, et plus particulièrement entre 50°C et 250°C, et la pression étant comprise entre la pression atmosphérique et 4 MPa.

7. Application du procédé de séparation par adsorption selon la revendication 1 à la purification d'un flux d'hydrocarbures contenant des impuretés soufrées et/ou azotées, la teneur en composés soufrés et/ou azotés étant inférieure à 500 ppm, et de manière préférée inférieure à 50 ppm, la température pendant la phase d'adsorption étant comprise entre 20°C et 400°C, de préférence entre 100°C et 280°C, et plus particulièrement entre 150°C et 250°C, et la pression étant comprise entre 0,3 et 15 MPa.

## Claims

1. A simulated moving bed process for adsorption separation of a molecular species from a mixture containing said species and other molecular species in any proportion, comprising bringing the mixture into contact with a solid adsorbent, the adsorbent being **characterized in that** it contains a solid with a crystalline structure analogous to that of IM-12 and having a chemical composition expressed, as the anhydrous base and in terms of moles of oxide, by the formula: XO₂: mYO₂: pZ₂O₃: qR_{2/n}O, in which R represents one or more cations with valency n, X represents one or more tetravalent elements other than germanium, Y represents germanium, and Z represents at least one trivalent element.

2. Application of an adsorption separation process according to claim 7 to the separation of a xylene isomer (ortho-, meta- or para-xylene) or ethylbenzene from a hydrocarbon feed essentially comprising C8 aromatic hydrocarbons, the desorbant employed being toluene, the volume ratio of the desorbant to the feed being in the range 0.5 to 2.5, preferably in the range 1 to 2, the temperature being in the range 20°C to 250°C, preferably in the range 90°C to 210°C, and more particularly in the range 160°C to 200°C, and the pressure being in the range from atmospheric pressure to 2 MPa.

3. Application of an adsorption separation process according to claim 1 to separating linear paraffins from any mixture of hydrocarbons containing them, said separation being carried out in the liquid phase, the temperature being in the range 100°C to 250°C, and the pressure being in the range 0.2 to 2 MPa, the desorbant used preferably being a hydrocarbon, in particular a C3-C6 paraffin or a mixture of C3-C6 paraffins.

4. Application of an adsorption separation process according to claim 1 to separating one or more isomers of dimethylnaphthalene from a hydrocarbon feed essentially comprising aromatic C12 hydrocarbons, said separation being carried out in a simulated moving bed, the preferred desorbant being toluene, the volume ratio of the desorbant to the feed being in the range 0.5 to 2.5, preferably in the range 1 to 2, the temperature generally being in the range 20°C to 300°C, preferably in the range 90°C to 260°C, and more particularly in the range 160°C to 250°C, and the pressure being in the range from atmospheric pressure to 2 MPa.

5. Application of an adsorption separation process according to claim 1 to separating one or more isomers of dichlorobenzene (ortho-, meta- or para-dichlorobenzene) from a feed essentially comprising dichlorobenzenes, said separation being carried out in a simulated moving bed, the preferred desorbant being toluene, para-xylene, meta-xylene or a mixture of xylenes, the temperature being in the range 20°C to 250°C, preferably in the range 90°C to 210°C, and more particularly in the range 120°C to 200°C, and the pressure being in the range from atmospheric pressure to 2 MPa.

6. Application of an adsorption separation process according to claim 1 to separating heavy aromatic compounds (polynuclear aromatics - PNA) present in hydrocracking residues, the temperature generally being in the range 20°C to 350°C, more particularly in the range 50°C to 250°C, and the pressure being in the range from atmospheric pressure to 4 MPa.

7. Application of an adsorption separation process according to claim 1 to purifying a stream of hydrocarbons containing sulphur-containing and/or nitrogen-containing impurities, the amount of sulphur-containing and/or nitrogen-containing compounds being less than 500 ppm, preferably less than 50 ppm, the temperature during the adsorption phase being in the range 20°C to 400°C, preferably in the range 100°C to 280°C, more particularly in the range 150°C to 250°C, and the pressure being in the range 0.3 to 15 MPa.

## Patentansprüche

1. Verfahren zum Trennen durch Adsorption im simulierten Bewegtbett einer Molekülspezies aus einem Kohlenwasserstoffgemisch, das die Spezies und andere Molekülspezies in einem beliebigen Anteil enthält, das ein Inkontaktbringen des Gemischs mit einem adsorbierenden Feststoff umfasst, wobei das Adsorptionsmittel **dadurch gekennzeichnet ist, dass** es einen Feststoff mit einer Kristallstruktur enthält, die analog zu der von IM-12 ist, und eine chemische Zusammensetzung aufweist, ausgedrückt auf einer wasserfreien Basis und in Oxidmol durch die Formel XO₂ : m YO₂: p Z₂O₃ : q R₂/ₙO, worin R für ein oder mehrere Kation(en) mit Valenz n steht, X für ein oder mehrere vierwertige(s) Element(e) steht, das (die) nicht Germanium ist (sind), Y für Germanium steht, und Z für mindestens ein dreiwertiges Element steht.

2. Anwendung des Verfahrens zum Trennen durch Adsorption nach Anspruch 1 auf das Trennen eines der Isomere von Xylol (Ortho-, Meta-oder Para-Xylol) oder von Ethylbenzen, aus einer Kohlenwasserstoffbeschickung, die im Wesentlichen aromatische Kohlenwasserstoffe mit 8 C-Atomen umfasst, wobei das verwendete Desorptionsmittel Toluol ist und das Volumenverhältnis von Desorptionsmittel zu Beschickung im Bereich zwischen 0,5 und 2,5 und bevorzugt zwischen 1 und 2 liegt, die Temperatur im Bereich zwischen 20°C und 250 °C, bevorzugt zwischen 90 °C und 210 °C und insbesondere zwischen 160 °C und 200 °C liegt, und der Druck im Bereich zwischen dem Atmosphärendruck und 2 MPa liegt.

3. Anwendung des Verfahrens zum Trennen durch Adsorption nach Anspruch 1 auf das Trennen der linearen Paraffine aus einem beliebigen Kohlenwasserstoffgemisch, das sie enthält, wobei das Trennen in flüssiger Phase ausgeführt wird, wobei die Temperatur im Bereich zwischen 100 °C und 250 °C liegt und wobei der Druck im Bereich zwischen 0,2 und 2 MPa liegt, wobei das verwendete Desorptionsmittel vorzugsweise ein Kohlenwasserstoff und insbesondere ein C3-C6-Paraffin oder ein Gemisch aus C3-C6-Paraffinen ist.

4. Anwendung des Verfahrens zum Trennen durch Adsorption nach Anspruch 1 auf das Trennen eines oder mehrerer der Isomere von Dimethylnaphtalen aus einer Kohlenwasserstoffbeschickung, die im Wesentlichen aromatische Kohlenwasserstoffe mit 12 C-Atomen umfasst, wobei das bevorzugte Desorptionsmittel Toluol ist, wobei das Volumenverhältnis von Desorptionsmittel zu Beschickung im Bereich zwischen 0,5 und 2,5 und bevorzugt zwischen 1 und 2 liegt, wobei die Temperatur im Allgemeinen im Bereich zwischen 20 °C und 300 °C, bevorzugt zwischen 90 °C und 260 °C und insbesondere zwischen 160 °C und 250 °C liegt, und wobei der Druck im Bereich zwischen dem Atmosphärendruck und 2 MPa liegt.

5. Anwendung des Verfahrens zum Trennen durch Adsorption nach Anspruch 1 auf das Trennen eines der Isomere von Dichlorbenzol (Ortho-, Meta- oder Para-Dichlorbenzol) aus einer Beschickung, die im Wesentlichen Dichlorbenzole umfasst, wobei das bevorzugte Desorptionsmittel Toluol, Para-Xylol, Meta-Xylol oder ein Gemisch aus Xylolen ist, wobei die Temperatur im Bereich zwischen 20 °C und 250 °C, bevorzugt zwischen 90 °C und 210 °C und insbesondere zwischen 120 °C und 200 °C liegt, und wobei der Druck im Bereich zwischen dem Atmosphärendruck und 2 MPa liegt.

6. Anwendung des Verfahrens zum Trennen durch Adsorption nach Anspruch 1 auf das Trennen der schweren aromatischen Verbindungen ("polynuclear aromatics" oder PNA) die in den Hydrocrackingrückständen vorhanden sind, wobei die Temperatur im Allgemeinen im Bereich zwischen 20 °C und 350 °C und insbesondere zwischen 50 °C und 250 °C liegt, und wobei der Druck im Bereich zwischen dem Atmosphärendruck und 4 MPa liegt.

7. Anwendung des Verfahrens zum Trennen durch Adsorption nach Anspruch 1 auf die Reinigung eines Kohlenwasserstoffstroms, der schwefelhaltige und/oder stickstoffhaltige Verunreinigungen enthält, wobei der Gehalt an schwefelhaltigen und/oder stickstoffhaltigen Verbindungen kleiner als 500 ppm und bevorzugt kleiner als 50 ppm ist, wobei die Temperatur während der Adsorptionsphase im Bereich zwischen 20 °C und 400 °C, bevorzugt zwischen 100 °C und 280 °C und insbesondere zwischen 150 °C und 250 °C liegt, und wobei der Druck im Bereich zwischen 0,3 und 15 MPa liegt.
